# EUROPÄISCHE PATENTSCHRIFT

(11) **EP 0 888 548 B1**
(45) Veröffentlichungstag und Bekanntmachung des Hinweises auf die Patenterteilung: **05.06.2002**
(21) Anmeldenummer: 97914229.6
(22) Anmeldetag: 12.03.1997
(51) Int. Cl.: G01N 33/558, G01N 33/58

(54) **VERFAHREN UND TESTSTREIFEN ZUR BESTIMMUNG EINES ANALYTEN**
METHOD AND TEST STRIP FOR DETERMINING AN ANALYTE
PROCEDE ET BANDE D'ESSAI POUR DETERMINER LA PRESENCE D'UN ANALYTE

(30) Priorität: 13.03.1996 DE 19609838
(43) Veröffentlichungstag der Anmeldung: 07.01.1999
(73) Patentinhaber: Roche Diagnostics GmbH, 68298 Mannheim (DE)
(72) Erfinder: BAUSBACK, Jörg, D-69151 Neckargemünd (DE)
(86) Internationale Anmeldenummer: EP9701253
(87) Internationale Veröffentlichungsnummer: WO9734147

(56) Entgegenhaltungen:
- EP-A- 0 362 809
- EP-A- 0 381 173
- EP-A- 0 585 912

## Beschreibung

Die Erfindung betrifft ein Bestimmungsverfahren für einen Analyten auf einem chromatographiefähigen Teststreifen unter Benutzung von partikel-markierten Bindungspartnern und einen entsprechenden Teststreifen.

Zum Nachweis von Analyten mit Hilfe analytspezifischer Bindungspartner werden seit einiger Zeit in zunehmendem Maße auch chromatographische Analysenelement oder Teststreifen eingesetzt. Insbesondere bei Immunoassays haben sich diese Testelemente bewährt, da aufgrund der chromatographischen Wanderung der Analytlösung schnell eine sog. "bound / free" Trennung stattfinden kann. Hierzu werden Anteile eines markierten Bindungspartners direkt oder indirekt beim Passieren eines Fangreagenzes in Abhängigkeit von der Menge des Analyten gebunden und können so bestimmt werden. Solche Teststreifen und Bestimmungsverfahren sind unter anderem in EP-A-0 186 799, EP-A-0 291 194, EP-A-0 323 605 und EP-A-0 250 137 beschrieben.

In jüngster Zeit wird zur Detektion zunehmend Gebrauch von sogenannten direktmarkierten Bindungspartnern gemacht, da deren Markierung im Gegensatz zu enzymmarkierten Bindungspartnern direkt ohne Zugabe eines Enzymsubstrates nachgewiesen werden kann. Dabei sind Markierungen vorteilhaft, die mit dem bloßen Auge sichtbar sind, insbesondere partikuläre, unlösliche Label, zum Beispiel gefärbter Latex, Farbsole und Metallsole, insbesondere Gold. In chromatographischen Immunoassays werden diese Direktmarkierungen bei Anwesenheit des Analyten in einer Abfangzone durch immobilisierte Bindungspartner fixiert und als Maß für die Anwesenheit des Analyten visuell gemessen. Dabei sind die Abfangreagenzien bevorzugt strichförmig auf kleinem Raum untergebracht, um eine höhere Konzentrierung der direktmarkierten Bindungspartner zu erreichen. Bei Anwesenheit eines Analyten bildet sich so in der strichförmigen Abfangzone ein gefärbter Strich, der mit dem bloßen Auge wahrgenommen werden kann.

Es hat sich herausgestellt, daß es dem menschlichen Auge, insbesondere bei kleinen Analytmengen, schwerfällt zu unterscheiden, ob sich in der Abfangzone ein Strich gebildet hat oder nicht. Insbesondere bei schlechten Lichtverhältnissen wird eine Färbung in der Abfangzone nicht bemerkt und so ein falsch-negatives Ergebnis abgelesen.

Aufgabe der Erfindung war es daher, ein Verfahren und ein Analyseelement zur Verfügung zu stellen, mit dem eine sicherere visuelle Ablesung auf einem Chromatographietestelement bei Benutzung von visuell detektierbaren, partikel-markierten Bindungspartnern insbesondere unter ungenügenden Lichtverhältnissen und / oder bei kleinen Analytkonzentrationen möglich ist. Auf der anderen Seite sollte das Verfahren die Ableseergebnisse bei normalem Tageslicht nicht negativ beeinflussen oder storen. Die Aufgabe wird gelöst durch die Erfindung, wie sie in den Ansprüchen aufgefuhrt ist.

Gegenstand der Erfindung ist ein Verfahren zur immunologischen Besrimmung eines Analyten auf einem chromatographiefähigen Teststreifen, enthaltend eine oder mehrere saugfähige Matrices auf einem Trägermaterial in flüssigkeitsübertragendem Kontakt zueinander, wobei die Matrices eine Aufgabezone an einem Ende und eine Saugzone am anderen Ende des Trägermaterials, eine Konjugatzone in der Aufgabezone oder in Anschluß an die Aufgabezone, die einen visuell detektierbaren, partikel-markierten Analytbindungspartner enthält, eine Chromatographiezone im Anschluß an die Konjugatzone und eine Abfangzone zwischen Chromatographiezone und Saugzone bilden, wobei die Abfangzone festphasengebundene Bindungspartner für den Analyten oder für einen unmarkierten analytspezifischen Bindungspartner enthält
durch Aufgabe der Analytlosung auf die Aufgabezone und Messung der in der Abfangzone gebundenen Markierung als Maß fiir den Analyten,
dadurch gekennzeichnet, daß auf die Aufgabezone oder auf eine Matrix zwischen Aufgabezone und Abfangzone ein Fluoreszenzfarbstoff aufgebracht wird, der fähig ist, in der Analytlösung durch die Abfangzone chromatographisch zu wandern
und daß die Anwesenheit der direktmarkierten Bindungspartner in der Abfangzone visuell unter gleichzeitiger Anregung des sich in dem Bereich der Abfangzone befindenden Fluoreszenzfarbstoffes aufgrund des entstehenden Kontrastes gemessen wird.

Weiterer Gegenstand der Erfindung ist ein chromatographiefähiger Teststreifen, enthaltend einen oder mehrere saugfahige Matrices auf einem Trägermaterial in flüssigkeitsübertragenden Kontakt zueinander, wobei die Matrices eine Aufgabezone an einem Ende und eine Saugzone am anderen Ende des Trägermaterials, eine Konjugatzone in der Aufgabezone oder in Anschluß an die Aufgabezone, die einen visuell detektierbaren, partikel-markierten Analytbindungspartner enthält, eine Chromatographiezone im Anschluß an die Konjugatzone und eine Abfangzone zwischen Chromatographiezone und Saugzone bilden, wobei die Abfangzone festphasengebundene Bindungspartner für den Analyten oder für einen unmarkierten anlytspezifischen Bindungspartner enthält,
dadurch gekennzeichnet, daß auf die Aufgabezone oder auf eine Matrix zwischen Aufgabezone und Abfangzone ein Fluoreszenzfarbstoff aufgebracht ist, der fähig ist, in der Analytlösung chromatographisch zu wandern.

Der Teststreifen enthält auf einem Trägermaterial eine oder mehrere saugfähige Matrices, die im wesentlichen nebeneinander angeordnet sind und die die verschiedenen Zonen bilden. Es ist möglich, daß mehrere Matrices aus gleichem oder unterschiedlichem Material in einer Zone vorliegen. Für die Aufgabezone und die Konjugatzone ist es möglich, daß diese hintereinander aus einem oder verschiedenen Matrixmaterialien bestehend angeordnet sind. Es ist aber auch möglich, daß Aufgabezone und Konjugatzone identisch sind und aus einer oder auch aus mehreren übereinander liegenden Matrices bestehen.

Unter Matrices werden kapillaraktive, saugfähige, vorzugsweise poröse Materialien verstanden, die faserig oder nicht-faserig sein können.

Als Trägermaterial kommen Schichten oder Streifen aus Glas, Metall oder Kunststoff in Frage. Streifen aus Kunststoffolien wie beispielsweise Polystyrol haben sich als besonders bevorzugt erwiesen.

Zur Befestigung der Matrices auf dem Trägermaterial ist es möglich, doppelseitiges Klebeband zu verwenden. Vorzugsweise wird jedoch Schmelzkleber eingesetzt.

Die Matrices sind auf dem Trägermaterial so angeordnet, daß sie eine Flüssigkeitstransportstrecke bilden und Flüssigkeit, die auf die Matrix oder die Matrices der Probenaufgabezone aufgegeben wird, durch Kapillarkrafte uber die Konjugatzone, die Chromatographiezone und die Abfangzone in die Saugzone gelangt. Hierzu ist es nötig, daß die verschiedenen Matrices miteinander in Flüssigkeitskontakt stehen. Dies kann dadurch erreicht sein, daß sich die Matrices über Kanten berühren. Vorteilhafterweise werden sich die Matrices jedoch teilweise überlappen.

Um eine für den Nachweis besonders niedrig konzentrierter Analyte ausreichende Flüssigkeitsmenge durch die Fangzone zu befördern, hat es sich als vorteilhaft erwiesen, wenn das sich an die Fangzone anschließende chromatographische Material in der Lage ist, möglichst viel der die Fangzone passierenden Flüssigkeit aufzunehmen. Besonders vorteilhaft ist es, wenn dieses Material (Saugzone) so viel Flüssigkeit aufnehmen kann, daß ein Großteil oder bevorzugt die gesamte Analytflüssigkeit die Abfangzone passieren kann.

Um niedrig konzentrierte Analyten nachweisen zu können, werden in dem Testträger spezifisch bindende Substanzen eingesetzt. Hierunter werden Bestandteile eines spezifischen Bindungspaares verstanden. Spezifische Bindungspaare sind beispielsweise Lektine und entsprechende Saccharide, DNA oder RNA und entsprechende komplementäre DNA oder RNA oder Antigene und an sie bindende Antikorper oder Antikörperfragmente. Auch Biotin oder Streptavidin sind ein spezifisches Bindungspaar. Im Rahmen der vorliegenden Erfindung wird auf die Verwendung von Antikörpern oder Antikorperfragmenten zur Erkennung von Antigenen besonders eingegangen. Eine Übertragung dieser Ausführungsformen auf andere spezifische Bindungspaare ist für den Fachmann jedoch leicht möglich.

In der Konjugatzone, die sich in der Probenaufgabezone befinden kann oder sich an diese anschließen kann, sind wanderungsfahige, partikelmarkierte analytspezifische Bindungspartner (Konjugate) imprägniert. In der vorliegenden Erfindung soll diese Markierung unter Normallicht visuell mit dem bloßen Auge erkennbar sein. Unter Partikeln als Markierung werden unlösliche Partikel, vorzugsweise der Größe 1-100 nm, verstanden. Besonders bevorzugt sind gefärbter Latex, gefärbte Liposomen, Selen, Telur, Ruß, Farbsole, Metallsole, ganz besonders bevorzugt ist Gold.

Die Menge des markierten analytspezifischen Bindepartners sollte vorzugsweise im Überschuß zu der zu erwartenden Analytmenge sein.

Als Konjugatmaterial haben sich saugfähige, vor allem poröse Kunststoffschichten oder Membranen als geeignet erwiesen, z.B. Papier, Vliese, poröse Kunststoffschichten oder Membranen. Als besonders geeignet haben sich faserige Matrices, die hohe Anteile an Glasfasern und / oder Synthetikfasern, wie beispielsweise Polyesterfasern und / oder Zellwolle enthalten, herausgestellt. Ganz besonders bevorzugt sind Vliese, wie sie in EP-A-0 326 135 beschrieben sind. Wen zwei oder mehrere Matrices im Probenaufgabebereich vorliegen, ist es bevorzugt, wenn sie im wesentlichen ubereinander liegen.

In der Abfangzone ist ein Abfangreagenz immobilisiert. Dies kann zum einen ein analytspezifischer Bindepartner direkt sein. Dies kann aber auch ein Bindereagenz für einen löslich auf dem Teststreifen auf der Aufgabezone oder zwischen Aufgabezone und Abfangzone aufgetragenen wanderungsfähigen unmarkierten Analytbindungspartner sein. Als bevorzugt hat es sich erwiesen, wenn das Abfangreagenz Streptavidin ist, das einen freibeweglich aufgebrachten biotinylierten Analytbindungspartner beim Passieren der Streptavidinzone binden kann.

Das Abfangreagenz kann nach dem Fachmann bekannten Methoden, z.B. kovalent immobilisiert sein (siehe z.B. EP-A-0 374 778). Als Matrix der Abfangzone hat sich auch Cellulosenitrat oder Nitrocelluloseester bewahrt, da hieran sehr viele Substanzen, z.B. Proteine oder Nukleinsäuren so stark absorptiv gebunden werden, daß eine chemische Bindung zur Immobilisierung nicht erforderlich ist.

Um eine möglichst hohe Farbdichte der in der Abfangzone abgefangenen markierten Bindepartner zu erreichen, ist es besonders vorteilhaft, das Abfangreagenz in hoher Dichte in einer möglichst kleinen Dimension zur Chromatographierichtung aufzubringen. Deshalb ist es bevorzugt, das Abfangreagenz als dunnen Strich oder Band senkrecht zur Teststreifenlängsrichtung in der Abfangzone zu immobilisieren.

Striche von mehreren Zehntel mm bis mehrere mm, vorzugsweise 0,5 - 1 mm Breite haben sich für den Nachweis von Analyten in Konzentrationen von etwa 10⁻¹² mol /l als sehr gut geeignet erwiesen. Das Abfangreagenz kann durch Strichdosierung mittels Kanüle, durch Ink-Jet oder Airbrush aufgebracht werden.

Dabei ist es vorteilhaft, wenn die Menge an Abfangreagenz wenigstens 1 µg / cm², ganz besonders bevorzugt bis 40 µg / cm² beträgt.

Erfindungsgemäß liegt nun bei oder vor der Analytaufgabe auf die Probenaufgabezone ein Fluoreszenzfarbstoffin der Aufgabezone oder zwischen Aufgabezone und Abfangzone löslich imprägniert vor. Dieser Fluoreszenzfarbstoff sollte durch externe Lichtquellen, z.B. unter einer UV-Lampe, leicht anregbar sein und bevorzugt im sichtbaren emittieren. Weiterhin muß der Fluroeszenzfarbstoff zusammen mit der Analytlösung auf dem Teststreifen chromatographieren. Falls der Fluoreszenzfarbstoff nicht mit der Analytlösung zusammen aufgegeben wird, ist es vorteilhaft, ihn über einen größeren Bereich senkrecht zur Chromatographierichtung aufzubringen, so daß er nach Abschluß der Chromatographie der Analytlösung mindestens einen Bereich vor und nach der Abfangzone bedeckt. Der Ort zwischen Aufgabezone und Abfangzone, auf dem der Fluoreszenzfarbstoff flächig aufgetragen ist, sollte so gewählt werden, daß der Fluoreszenzfarbstoff in der Analytflüssigkeit so mitchromatographiert, daß nach Abschluß der Chromatographie Fluroeszenzfarbstoff zu beiden Seiten der Abfangzone sich befindet. Der Fachmann kann ohne großen Aufwand je nach Löslichkeit und Chromatographieverhalten eines gewählten Fluoreszenzfarbstoffes den Imprägnierungsort und die Imprägnierungsfläche auf einer der Teststreifenmatrices vor der Abfangzone festlegen.

Als Fluoreszenzfarbstoffe besonders geeignet sind AMF (Aminomethylfluorescein), 5(6)-Carboxyfluorescein-N-hydroxy-succinimidester (Fluos), Acridinorange, Texasrot, Rhodamin oder Resorufin.

Der Fluoreszenzfarbstoff sollte bei Tageslicht keine oder relativ geringe Färbung zeigen oder gegebenenfalls eine von der Farbe der Direktmarkierung unterschiedliche Färbung. Im Unterschied zum direktmarkierten Konjugat liegt der Fluoreszenzfarbstoff nicht an immunologischen Bindungspartnern konjugiert vor. Ferner darf der Fluoreszenzfarbstoff nicht die immunologische Reaktion beeinflussen.

Zur Durchführung des Immunoassays wird auf die Probenaufgabezone Probenflüssigkeit aufgegeben. Die Probenflüssigkeit kann mit dem Fluroeszenzfarbstoff vermischt werden. Bevorzugt ist der Fluroeszenzfarbstoff aber vor Aufgabe der Probenflüssigkeit auf dem Teststreifen imprägniert.

Bei einem Sandwich-Immunoassay bildet der Analyt im Laufe der Chromatographie der Analytlösung zur Abfangzone einen Sandwich-Komplex mit dem markierten Analytbindungspartner und einem zweiten, nicht markierten Analytbindungspartner. Dieser ist entweder in der Abfangzone immobilisiert oder ist, versehen mit einer spezifischen Bindungsstelle für ein Fangreagenz der Abfangzone, zwischen Aufgabezone und Abfangzone löslich imprägniert. Nicht analytgebundene, markierte Bindungspartner passieren die Abfangzone, während über Analyt gebundene markierte Bindungspartner in der Abfangzone gebunden werden und durch ihre Färbung die Anwesenheit von Analyt anzeigen.

Mit der Analytlösung chromatographiert erfindungsgemäß in der Analytlösung gelöster Fluoreszenzfarbstoff mindestens soweit, daß ein Teil des Fluoreszenzfarbstoffes, aber nicht die gesamte Menge des Fluoreszenzfarbstoffes die Abfangzone passiert und so die Abfangzone nach Ende der Chromatographie beidseitig umgibt.

Bei Bestrahlen der Abfangzone mit Licht geeigneter Fluoreszenz-Anregungswellenlänge, zum Beispiel UV-Licht, hat es sich überraschenderweise herausgestellt, daß bei Anwesenheit von Analyt die an die Abfangzone gebundene nicht-fluoreszierende Partikelmarkierung sehr stark gegen den durch Fluoreszenzfarbstoff gebildeten fluoreszierenden Hintergrund hervortritt, z.B. als schwarzer Strich. Bei geringen Analytmengen können so insbesondere bei ungenügenden Lichtverhältnissen durch einfache Anstrahlung des Teststreifens mit einer Fluoreszenzlampe die Anwesenheit weit geringerer Mengen an festphasengebundener Markierung mit dem bloßen Auge erkannt werden, als dies ohne diese Fluoreszenz-Kontrastverstärkung möglich wäre.

Auf der anderen Seite war es uberraschend, daß es bei Abwesenheit von Analyt zu keinerlei - eventuell zu falsch positiven Resultaten führenden - Kontrastunterschieden zwischen der Abfangzone und dem sich vor und danach anschließenden Fluoreszenzhintergrund kommt. Die Abfangzone mit dem unmarkierten festphasengebundenen Analytbindungspartner erscheint unter Fluoreszenzlicht zusammen mit dem Bereich davor und danach bei Abwesenheit von Analyt einheitlich fluoreszierend.

Ein weiterer Vorteil der erfindungsgemäßen Kontrastverstärkung ist, daß bei einer visuellen Detektion der Abfangzone bei Normallicht sich keinerlei störende Einflüsse durch den Fluoreszenzfarbstoff ergeben. Das erfindungsgemäße Analyseverfahren kann demnach wahlweise entsprechend dem Stand der Technik ohne Qualitätseinbuße ohne Fluoreszenzanregung (falls zum Beispiels keine UV-Lampe zur Verfügung steht) oder entsprechend der Erfindung mit Kontrastverstärkung durch Fluoreszenzanregung durchgeführt werden.

Das erfindungsgemäße Verfahren ermoglicht es, die Abwesenheit mit sehr niedrig konzentrierte Analyte in der Größenordnung von 10⁻¹⁰ - 10⁻¹² mol / l nachzuweisen.

## Patentansprüche

1. Verfahren zur immunologischen Bestimmung eines Analyten auf einem chromatographiefähigen Teststreifen, enthaltend eine oder mehrere saugfähige Matrices auf einem Trägermaterial in flussigkeitsubertragendem Kontakt zueinander, wobei die Matrices eine Aufgabezone an einem Ende und eine Saugzone am anderen Ende des Trägermaterials, eine Konjugatzone in der Aufgabezone oder in Anschluß an die Aufgabezone, die einen visuell detektierbaren, partikel-markierten Analytbindungspartner enthält, eine Chromatographiezone im Anschluß an die Konjugatzone und eine Abfangzone zwischen Chromatographiezone und Saugzone bilden, wobei die Abfangzone festphasengebundene Bindungspartner fiir den Analyten oder für einen unmarkierten analytspezifischen Bindungspartner enthält
durch Aufgabe der Analytlosung auf die Aufgabezone und Messung der in der Abfangzone gebundenen Markierung als Maß für den Analyten,
**dadurch gekennzeichnet, daß** auf die Aufgabezone oder auf eine Matrix zwischen Aufgabezone und Abfangzone ein Fluoreszenzfarbstoff aufgebracht wird, der fähig ist, in der Analytlösung durch die Abfangzone chromatographisch zu wandern
und daß die Anwesenheit der direktmarkierten Bindungspartner in der Abfangzone visuell unter gleichzeitiger Anregung des sich in dem Bereich der Abfangzone befindenden Fluoreszenzfarbstoffes gemessen wird.

2. Verfahren gemäß Anspruch 1, **dadurch gekennzeichnet, daß** in der Aufgabezone oder zwischen Aufgabezone und Abfangzone ein wanderungsfähiger, unmarkierter analytspezifischer Bindepartner mit einer Bindestelle für den festphasengebundenen Bindungspartner aufgebracht ist.

3. Verfahren gemäß Anspruch 1 oder 2, **dadurch gekennzeichnet, daß** die Abfangzone strichförmig ist.

4. Verfahren gemäß einem der Anspruche 1-3, **dadurch gekennzeichnet, daß** die Markierung eine Metallmarkierung ist.

5. Verfahren gemäß Anspruch 4, **dadurch gekennzeichnet, daß** die Markierung Gold ist.

6. Verfahren gemäß einem der Anspruche 1-5, **dadurch gekennzeichnet, daß** in der Abfangzone Streptavidin immobilisiert ist.

7. Verfahren gemäß einem der Anspruche 1-6, **dadurch gekennzeichnet, daß** der Fluoreszenzfarbstoff in der Aufgabezone imprägniert ist.

8. Verfahren gemäß Anspruch 1-6, **dadurch gekennzeichnet, daß** der Fluoreszenzfarbstoff in der Konjugatzone impragniert ist.

9. Verfahren gemäß einem der Anspruch 1-8, **dadurch gekennzeichnet, daß** die Saugzone soviel Flüssigkeit aufnimmt, daß alle markierten Konjugate die Abfangzone passieren können.

10. Chromatographiefähiger Teststreifen, enthaltend eine oder mehrere saugfähige Matrices auf einem Trägermaterial in flüssigkeitsübertragendem Kontakt zueinander, wobei die Matrices eine Aufgabezone an einem Ende und eine Saugzone am anderen Ende des Trägermaterials, eine Konjugatzone in der Aufgabezone oder in Anschluß an die Aufgabezone, die einen visuell detektierbaren, partikel-markierten Analytbindungspartner enthält, eine Chromatographiezone im Anschluß an die Konjugatzone und eine Abfangzone zwischen Chromatographiezone und Saugzone bilden, wobei die Abfangzone festphasengebundene Bindungspartner für den Analyten oder für einen unmarkierten analytspezifischen Bindungspartner enthält,
**dadurch gekennzeichnet, daß** auf die Aufgabezone oder auf eine Matrix zwischen Aufgabezone und Abfangzone ein Fluoreszenzfarbstoff aufgebracht ist, der fähig ist, in der Analytlösung chromatographisch zu wandern.

11. Teststreifen gemäß Anspruch 10, **dadurch gekennzeichnet, daß** in der Aufgabezone oder zwischen Aufgabezone und Abfangzone ein unmarkierter, wanderungsfähiger, analytspezifischer Bindepartner mit einer Bindungsstelle für den festphasengebundenen Bindungspartner untergebracht ist.

12. Teststreifen gemäß Anspruch 10 oder 11, **dadurch gekennzeichnet, daß** die Abfangzone strichförmig ist.

13. Teststreifen gemäß Anspruch 10-12, **dadurch gekennzeichnet, daß** die Markierung aus einer Metallsol besteht.

14. Teststreifen gemäß Anspruch 10-13, **dadurch gekennzeichnet, daß** die Markierung aus Gold besteht.

15. Teststreifen gemäß Anspruch 10-14, **dadurch gekennzeichnet, daß** in der Abfangzone Streptavidin immobilisiert ist.

16. Teststreifen gemäß Anspruch 10-15, **dadurch gekennzeichnet, daß** der Fluoreszenzfarbstoff in der Aufgabezone imprägniert ist.

17. Teststreifen gemäß Anspruch 10-15, **dadurch gekennzeichnet, daß** der Fluoreszenzfarbstoff in der Konjugatzone imprägniert ist.

18. Teststreifen gemäß Anspruch 10-17, **dadurch gekennzeichnet, daß** der Fluoreszenzfarbstoff Aminophenylfluoreszein ist.

## Claims

1. Method for the immunological determination of an analyte on a chromatographic test strip containing one or several absorbent matrices on a support material in liquid-transferring contact to one another, wherein the matrices form an application zone at one end and a suction zone at the other end of the support material, a conjugate zone in the application zone or adjoining the application zone which contains a visually detectable, particle-labelled analyte binding partner, a chromatographic zone adjoining the conjugate zone and a capture zone between the chromatographic zone and suction zone, wherein the capture zone contains solid phase-bound binding partner for the analyte or for an unlabelled analyte-specific binding partner
by applying the analyte solution to the application zone and measuring the label bound in the capture zone as a measure for the analyte,
**characterized in that** a fluorescent dye is applied to the application zone or to a matrix between the application zone and capture zone which is able to chromatographically migrate in the analyte solution through the capture zone
and that the presence of the directly labelled binding partner in the capture zone is measured visually while simultaneously exciting the fluorescent dye located in the area of the capture zone.

2. Method as claimed in claim 1, **characterized in that** an unlabelled analyte-specific binding partner that is able to migrate and contains a binding site for the solid phase-bound binding partner is applied in the application zone or between the application zone and capture zone.

3. Method as claimed in claim 1 or 2, **characterized in that** the capture zone is in the shape of a line.

4. Method as claimed in one of the claims 1-3, **characterized in that** the label is a metal label.

5. Method as claimed in claim 4, **characterized in that** the label is gold.

6. Method as claimed in one of the claims 1-5, **characterized in that** streptavidin is immobilized in the capture zone.

7. Method as claimed in one of the claims 1-6, **characterized in that** the fluorescent dye is impregnated in the application zone.

8. Method as claimed in claim 1-6, **characterized in that** the fluorescent dye is impregnated in the conjugate zone.

9. Method as claimed in one of the claims 1-8, **characterized in that** the suction zone takes up sufficient liquid to enable all labelled conjugates to pass through the capture zone.

10. Chromatographic test strip containing one or several absorbent matrices on a support material in liquid-transferring contact to one another, wherein the matrices form an application zone at one end and a suction zone at the other end of the support material, a conjugate zone in the application zone or adjoining the application zone which contains a visually detectable, particle-labelled analyte binding partner, a chromatographic zone adjoining the conjugate zone and a capture zone between the chromatographic zone and suction zone, wherein the capture zone contains solid phase-bound binding partner for the analyte or for an unlabelled analyte-specific binding partner
**characterized in that** a fluorescent dye is applied to the application zone or to a matrix between the application zone and capture zone which is able to chromatographically migrate in the analyte solution.

11. Test strip as claimed in claim 10, **characterized in that** an unlabelled analyte-specific binding partner that is able to migrate and contains a binding site for the solid phase-bound binding partner is located in the application zone or between the application zone and capture zone.

12. Test strip as claimed in claim 10 or 11, **characterized in that** the capture zone is in the shape of a line.

13. Test strip as claimed in claim 10-12, **characterized in that** the label is composed of a metal sol.

14. Test strip as claimed in claim 10-13, **characterized in that** the label is composed of gold.

15. Test strip as claimed in claim 10-14, **characterized in that** streptavidin is immobilized in the capture zone.

16. Test strip as claimed in claim 10-15, **characterized in that** the fluorescent dye is impregnated in the application zone.

17. Test strip as claimed in claim 10-15, **characterized in that** the fluorescent dye is impregnated in the conjugate zone.

18. Test strip as claimed in claim 10-17, **characterized in that** the fluorescent dye is aminophenyl fluorescein.

## Revendications

1. Procédé pour la détermination immunologique d'un analyte sur une bande de test permettant la chromatographie, contenant sur un matériau de support une ou plusieurs matrices absorbantes en contact mutuel de transfert de liquide, les matrices formant une zone d'application à une extrémité et une zone d'aspiration à l'autre extrémité du matériau de support, une zone de conjugaison dans la zone d'application ou se raccordant à la zone d'application et contenant un partenaire de liaison à l'analyte détectable visuellement et marqué par particules, une zone de chromatographie qui se raccorde à la zone de conjugaison et une zone d'interception entre la zone de chromatographie et la zone d'aspiration, la zone d'interception contenant, liés à une phase solide, des partenaires de liaison pour l'analyte ou pour un partenaire de liaison spécifique à l'analyte et non marqué, la détermination s'effectuant par application de la solution d'analyte sur la zone d'application et par mesure du marqueur lié dans la zone d'interception et servant de mesure pour l'analyte,
**caractérisé en ce qu'**un colorant fluorescent qui est en mesure de traverser chromatographiquement la zone d'interception dans la solution d'analyte est appliqué sur la zone d'application ou sur une matrice située entre la zone d'application et la zone d'interception, et **en ce que** la présence des partenaires de liaison marqués directement dans la zone d'interception est mesurée visuellement en excitant également le colorant fluorescent qui se trouve dans la région de la zone d'interception.

2. Procédé selon la revendication 1, **caractérisé en ce qu'**un partenaire de liaison apte à se déplacer, non marqué et spécifique à l'analyte, qui présente un site de liaison pour le partenaire de liaison lié à la phase solide, est appliqué dans la zone d'application ou entre la zone d'application et la zone d'interception.

3. Procédé selon la revendication 1 ou 2, **caractérisé en ce que** la zone d'interception est en forme de trait.

4. Procédé selon l'une des revendications 1 à 3, **caractérisé en ce que** le marqueur est un marqueur métallique.

5. Procédé selon la revendication 4, **caractérisé en ce que** le marqueur est de l'or.

6. Procédé selon l'une des revendications 1 à 5, **caractérisé en ce que** de la streptavidine est immobilisée dans la zone d'interception.

7. Procédé selon l'une des revendications 1 à 6, **caractérisé en ce que** le colorant fluorescent imprègne la zone d'application.

8. Procédé selon les revendications 1 à 6, **caractérisé en ce que** le colorant fluorescent imprègne la zone de conjugaison.

9. Procédé selon l'une des revendications 1 à 8, **caractérisé en ce que** la zone d'aspiration reprend suffisamment de liquide pour que tous les conjugués marqués puissent traverser la zone d'interception.

10. Bande de test permettant la chromatographie, contenant sur un matériau de support une ou plusieurs matrices absorbantes en contact mutuel de transfert de liquide, les matrices formant une zone d'application à une extrémité et une zone d'aspiration à l'autre extrémité du matériau de support, une zone de conjugaison dans la zone d'application ou se raccordant à la zone d'application et contenant un partenaire de liaison à l'analyte détectable visuellement et marqué par particules, une zone de chromatographie qui se raccorde à la zone de conjugaison et une zone d'interception entre la zone de chromatographie et la zone d'aspiration, la zone d'interception contenant, liés à une phase solide, des partenaires de liaison pour l'analyte ou pour un partenaire de liaison spécifique à l'analyte et non marqué,
**caractérisée en ce qu'**un colorant fluorescent qui est en mesure de se déplacer par chromatographie dans la solution d'analyte est appliqué sur la zone d'application ou sur une matrice située entre la zone d'application et la zone d'interception.

11. Bande de test selon la revendication 10, **caractérisée en ce qu'**un partenaire de liaison non marqué, apte à se déplacer et spécifique à l'analyte, qui présente un site de liaison pour le partenaire de liaison lié à la phase solide, est placé dans la zone d'application ou entre la zone d'application et la zone d'interception.

12. Bande de test selon la revendication 10 ou 11, **caractérisée en ce que** la zone d'interception est en forme de trait.

13. Bande de test selon les revendications 10 à 12, **caractérisée en ce que** le marqueur est constitué d'un colloïde de métal.

14. Bande de test selon les revendications 10 à 13, **caractérisée en ce que** le marqueur est constitué d'or.

15. Bande de test selon les revendications 10 à 14, **caractérisée en ce que** de la streptavidine est immobilisée dans la zone de l'interception.

16. Bande de test selon les revendications 10 à 15, **caractérisée en ce que** le colorant fluorescent imprègne la zone d'application.

17. Bande de test selon les revendications 10 à 15, **caractérisée en ce que** le colorant fluorescent imprègne la zone de conjugaison.

18. Bande de test selon les revendications 10 à 17, **caractérisée en ce que** le colorant fluorescent est l'aminophénylfluorescéine.
